(19) Europäisches Patentamt
European Patent Office
Office européen des brevets

(11) Publication number: **0 162 701 B1**

# (12) EUROPEAN PATENT SPECIFICATION

(45) Date of publication of patent specification: **09.08.89**

(21) Application number: **85303577.2**

(22) Date of filing: **21.05.85**

(51) Int. Cl.[4]: **C 07 H 17/04,** C 07 D 493/04 // (C07D493/04, 317:00, 307:00)

## (54) Process for producing etoposide and intermediate for use therein.

(30) Priority: **22.05.84 JP 101766/84**

(43) Date of publication of application: **27.11.85 Bulletin 85/48**

(45) Publication of the grant of the patent: **09.08.89 Bulletin 89/32**

(84) Designated Contracting States: **CH DE FR GB IT LI NL SE**

(56) References cited:
**EP-A-0 111 058**
**CHEMICAL ABSTRACTS, vol. 101, no. 3, July 1984, page 639, no. 23883h, Columbus, Ohio, US; & JP - A - 58 225 096 (NIPPON KAYAKU CO., LTD.) 27-12-1983**
**CHEMICAL ABSTRACTS, vol. 100, no. 21, May 1984, page 670, no. 175210d, Columbus, Ohio, US; & JP - A - 58 219 196 (NIPPON KAYAKU CO., LTD.) 20-12-1983**

**The file contains technical information submitted after the application was filed and not included in this specification**

(73) Proprietor: **NIPPON KAYAKU KABUSHIKI KAISHA
Tokyo Fujimi Bldg. 11-2 Fujimi 1-chome
Chiyoda-ku
Tokyo 102 (JP)**

(72) Inventor: **Fujii, Tadashi
2-304, 410-11 Kakura
Iwatsuki-shi Saitama-ken (JP)**
Inventor: **Chikui, Yukio
2-12-8-302 Akabane
Kita-ku Tokyo (JP)**

(74) Representative: **Woods, Geoffrey Corlett et al
J.A. KEMP & CO. 14 South Square Gray's Inn
London WC1R 5EU (GB)**

(56) References cited:
**Protective groups in Organic Synthesis. T.W. Greene, pages 55, 56, 88**

**EP 0 162 701 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

A process for producing etoposide represented by the formula:

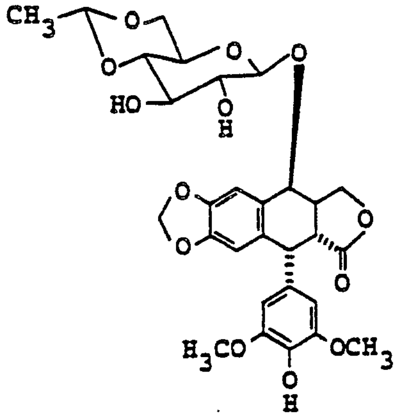

(I)

comprising the following steps (1) and (2) is already known (see Canadian Patent No. 956939):

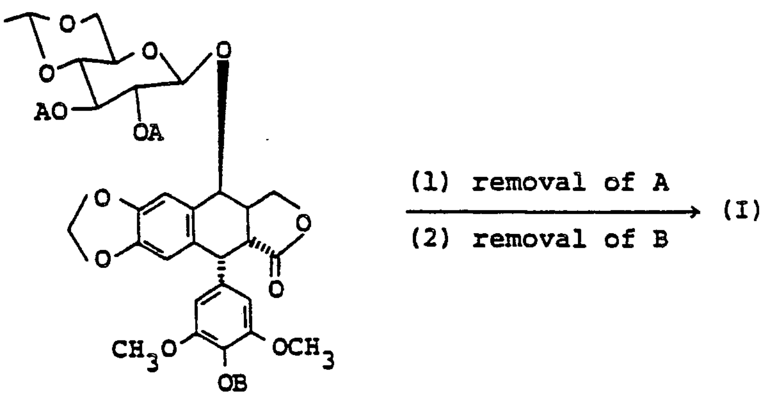


(II)

wherein A represents formyl or acetyl, and B represents benzyloxycarbonyl.

However, the above process has drawbacks that two steps, i.e., a step of removing A and a subsequent step of removing B, are necessary. The removal of A is time-consuming (for example, the reaction cannot be completed even after 20 to 30 hours) and, because of the increased production of by-products such as colored products, the quality of the produced etoposide is poor and its yield is low.

EP—A—111,058, having an earlier priority date than, but published after, the priority date of the present application discloses that etoposide may be prepared from the compound of formula (II) wherein A and B both represent —$COCH_2X$ wherein X is a halogen atom.

Chemical Abstracts 101:23883h (corresponding to JP—A—83/225,096) discloses that etoposide may be prepared by reducing the compound of formula (II) wherein A and B both represent —$CO_2CH_2CCl_3$ with zinc in AcOH/dioxane.

The present invention provides a process for producing etoposide represented by the formula (I):

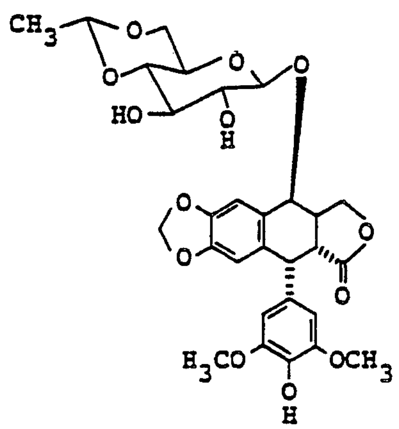

which comprises reacting a 4'-halogenoacetyl-4'-demethylepipodophyllotoxin-β-D-2,3-di-O-halogeno-acetyl-4,6-O-ethylideneglucoside represented by the general formula:

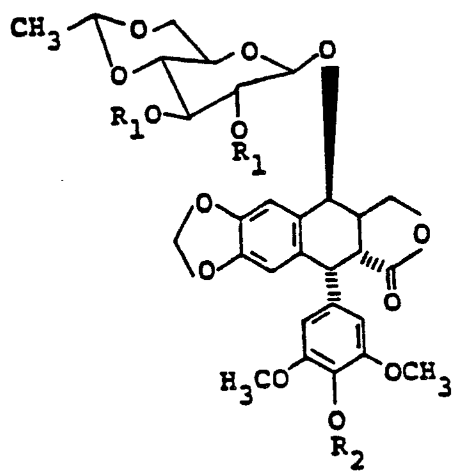

(III)

wherein $R_1$ and $R_2$, which may be the same or different, each represent $—COCHCl_2$ or $—COCCl_3$

(a) with an alcohol in the presence of a tri($C_1$—$C_4$ alkyl)amine, a pyridine or an organic carboxylic acid salt or

(b) with an amine and/or ammonia in an alcohol solvent, thereby to remove the halogenoacetyl groups so as to obtain etoposide of the formula (I).

$R_1$ and $R_2$ can be removed simultaneously to obtain etoposide and the obtained etoposide has a low impurity content so that it can be easily purified.

As examples of the alcohols which can be used in this invention, there can be mentioned $C_1$—$C_4$ alcohols having from 1 to 3 hydroxyl groups, and aminoalcohols having from 1 to 3 $C_1$—$C_4$ hydroxyalkyl groups banded to the nitrogen atom thereof, more particularly, $C_1$—$C_4$ monohydric alcohols such as methanol, ethanol, propanol, and butanol, $C_1$—$C_4$ polyhydric alcohols such as ethylene glycol and glycerine, and monoethanolamine, dimethanolamine and tripropanolamine, among which $C_1$—$C_4$ monohydric alcohols such as methanol and ethanol are desirable. It is desirable to use these alcohols as solvents for the present reaction, but alcohols other than these may be used. In this case, it is usual that the alcohol is used in an amount at least equivalent to that of the compound of formula (III).

As examples of the amines which can be used in the present invention, there are ($C_1$—$C_6$ alkyl) primary amines such as methylamine, ethylamine, n-propylamine, and n-butylamine; ($C_1$—$C_6$ alkyl) secondary amines such as dimethylamine, diethylamine, di-n-propylamine, di-n-butylamine, and di-n-hexylamine; $C_4$—$C_5$ cyclic amines which may contain at least one oxygen atom, such as pyrrolidine, piperidine or morpholine, and aliphatic diamines such as ethylenediamine, among which the $C_1$—$C_4$ alkyl primary and secondary amines such as methylamine and diethylamine are desirable.

The amine and/or ammonia is used in an alcohol solvent. An at least equivalent amount, preferably from 1 to 3 equivalents, of said amine or ammonia is usually used per equivalent of the used compound of formula (III).

Although it is of course possible to add an amine or ammonia as such to the reaction system, it is also possible that an acetate or hydrochloride of an amine and/or ammonia is added in the presence of a base, for example pyridine or triethylamine, to the reaction system and to effect the reaction with the free amine and/or ammonia prepared in situ.

In the present invention, the amines, or ammonia may be used alone in the alcohol solvent or as a mixture of at least two of them.

The tri($C_1$—$C_4$ alkyl)amine, pyridine or organic carboxylic acid is a catalyst. Examples of the tri($C_1$—$C_4$ alkyl)amine are trimethylamine and triethylamine. Examples of the pyridine are pyridine itself or a $C_1$—$C_4$ alkyl-substituted pyridine. As the organic carboxylic acid salts, there can be mentioned:

(1) metal salts or ammonium salts of an at least monobasic aliphatic carboxylic acid, for example sodium acetate, potassium acetate, magnesium acetate, sodium propionate, sodium succinate, ammonium formate, ammonium acetate, ammonium malonate, ammonium succinate and alkylammonium acetate;

(2) metal salts (for example alkali metal salts or alkaline earth metal salts) or ammonium salts of aromatic carboxylic acids, for example sodium benzoate, ammonium isonicotinate, ammonium benzoate, ammonium anthranilate and alkylammonium benzoate; and

(3) ammonium salt- or metal salt- (for example alkali metal salt- or alkaline earth metal salt-) weakly acidic cation exchange resins having carboxyl groups as exchangeable groups. Ammonium salts are preferred, desirably $C_1$—$C_3$ saturated fatty acid ammonium salts and, more desirably, ammonium acetate

and ammonium formate. The organic carboxylic acid salt is generally present in an amount of from 5 to 100 w/w %, more desirably 30 to 50 w/w %, based on the compound of formula (III).

The reaction temperature varies with the solvent or catalyst used, but it is usually from −10 to 100°C, desirably from 0 to 90°C, and especially from 20 to 70°C. The reaction is completed within 0.1 to 7 hours.

The compound of formula (III) is novel and can be synthesized by reacting using well-known 4′-demethylepipodophyllotoxin of formula (IV) (see U.S. Patent No. 3524844)

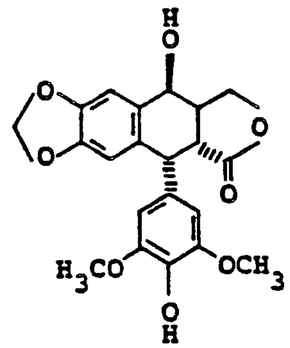

(IV)

with a dichloro- or trichloro-acetyl halide in an inert solvent, and condensing the resulting 4′-chloroacetyl-4′-demethylepipodophyllotoxin of the formula (V):

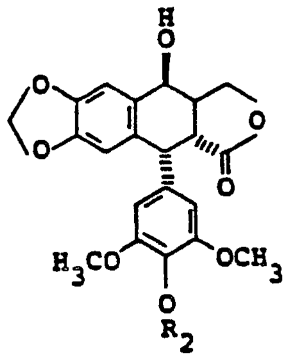

(V).

wherein $R_1$ represents $-COCHCl_2$ or $-COCCl_3$ with a 4,6-O-ethylidene-2,3-di-O-halogenoacetyl-β-D-glucopyranose of the formula (VI):

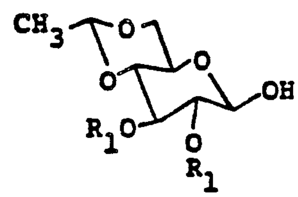

(VI)

(VI)

wherein $R_1$ represents $-COCHCl_2$ or $-COCCl_3$ at a temperature lower than 0°C in an inert solvent and in the presence of boron trifluoride ethyl etherate.

A preferred dichloro- or trichloro-acetyl halide is the dichloro- or trichloro-acetyl chloride.

The compound of formula (VI) is novel and can be synthesized from the well-known 4,6-O-ethylidene-1-O-benzyloxycarbonyl-β-D-glucopyranose of formula (VII) through, for example, the following reaction route:

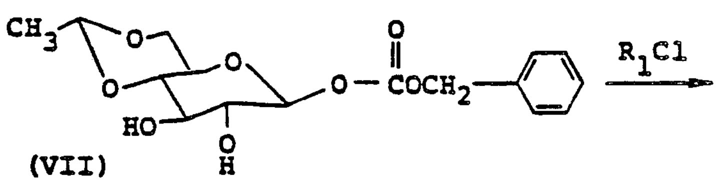


(VII)

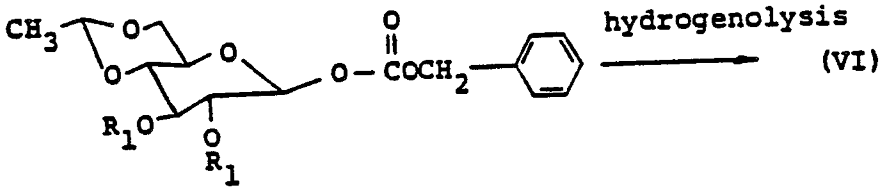

(VI)

(VIII)

wherein $R_1$ is as defined above.

Thus the compound of formula (VI) can be obtained by reacting 4,6-O-ethylidene-1-O-benzyloxycarbonyl-β-D-glucopyranose of formula (VII) with a chloro- or trichloroacetyl chloride in an inert solvent and subjecting the resulting 4,6-O-ethylidene-1-O-benzyloxycarbonyl-2,3-di-O-halogeno-acetyl-β-D-glucopyranose of formula (VIII) to hydrogenolysis. Although the formation of a small amount of an α-isomer of the compound of formula (VI) is not avoidable in the hydrogenolysis, the separation of the α-isomer from the β-isomer is easy since the β-isomer can be crystallized selectively from the reaction solution. Further, the β-isomer of the compound of formula (VI) has good stability, and undergoes substantially no isomerization into the α-isomer, so that it can be stored for a long time.

According to the present invention, the removal of the chloroacetyl groups can be effected under gentle conditions, for example, at 25°C or room temperature, within a short time, so that there is little formation of by-products such as colored products. It is possible to obtain etoposide from the compound of (III) in high yields. Therefore, purification after the reaction such as removal of colored products is easy and, for example, pure etoposide can be obtained by adding a hydrophobic solvent such as chloroform to the reaction solution, washing the solution with water, distilling off the solvent and recrystallizing the residue from a solvent. The resulting etoposide has such an extremely low inorganic content that its ignition residue (the residue left after ignition of the etoposide) is less than or equal to 0.1% by weight. Furthermore, when the reaction occurs in the presence of the alcohol with an ammonium salt of a lower fatty acid, such as ammonium acetate, or a tertiary amine, or when the reaction occurs with the amine and/or ammonia in an alcohol solvent, the reaction is completed within a short time at room temperature, and the etoposide can be recovered merely by concentrating the reaction solution, so that the reaction operation and the after-treatment subsequent to the reaction are easy. This makes the process extremely advantageous as an industrial production process, particularly as the reaction can be brought to completion within a short time.

The present invention will now be described in detail with reference to examples.

Example 1

1 g of 4′-dichloroacetyl-4′-demethylepipodophyllotoxin-β-D-2,3-di-O-dichloroacetyl-4,6-O-ethylidene-glucoside (III) ($R_1$, $R_2$ = —$COCHCl_2$) and 1 g of ammonium acetate were dissolved in 20 ml of methanol, and the solution was stirred at 25°C for 1.5 hours. After completion of the reaction, the methanol was concentrated to 10 ml and the resulting solution was cooled to obtain 0.55 g (yield: 86.1%) of etoposide.

The $R_f$ value of TLC (silica gel, developer, chloroform:methanol (1:1)), IR, NMR, and optical rotation of the obtained crystal were identical to those of the substance obtained by the process of Canadian Patent No. 956939.

m.p. 259—260°C, $R_f$ = 0.44.

Examples 2 to 9

1 g of compound (III) ($R_1$, $R_2$ = —$COCHCl_2$) was reacted under conditions shown in the following table, and the reaction solution was treated in the same manner as in Example 1 to obtain etoposide.

| Ex-ample | Catalyst | | Amount of methanol | Reaction temperature Reaction time | Yield of etoposide (I) |
|---|---|---|---|---|---|
| 2 | $HCO_2NH_4$ | 1 g | 20 ml | 25°C 3 hr | 86.5 % |
| 3 | $H_2CCO_2NH_4$<br>$H_2CCO_2NH_4$ | 1 g | 20 ml | " 7 hr | 77.8 % |
| 4 | 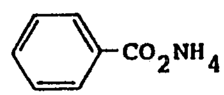 | 1 g | 20 ml | " 4 hr | 82.4 % |
| 5 | 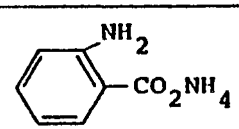 | 1 g | 20 ml | " 4 hr | 81.5 % |
| 6 | 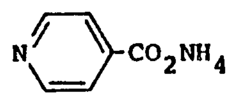 | 1 g | 20 ml | " 4 hr | 81.8 % |
| 7 | 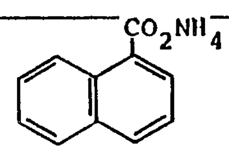 | 1 g | 20 ml | " 2 hr | 83.1 % |

| Ex-ample | Catalyst | Amount of methanol | Reaction temperature Reaction time | Yield of etoposide (I) |
|---|---|---|---|---|
| 8 | 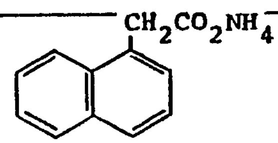 1 g | 20 ml | 25°C 2 hr | 80.2 % |
| 9 | weakly acidic ion exchange resin* 1 g | 20 ml | " 5 hr | 78.6 % |

*WK-20® ($N^+H_4$): a product of Mitsubishi Chemical Industries, Ltd.

Example 10

1 g of 4'-dichloroacetyl-4'-demethylepipodophyllotoxin-β-D-2,3-di-O-dichloroacetyl-4,6-O-ethylideneglucoside (III) ($R_1$, $R_2$ = —$COCHCl_2$) and 1 g of magnesium acetate were refluxed for 4 hours in 20 ml of methanol. After completion of the reaction, the methanol was distilled off and, after adding 30 ml of chloroform, the residue was washed with water and dried over anhydrous magnesium sulfate. The solvent was distilled off in vacuum, and the residue was recrystallized from methanol to obtain 0.49 g (yield: 76.7%) of etoposide crystals.

Comparative Example 1

4.75 g (yield: 64.6%) of etoposide crystals were obtained by the same reaction as in Example 1 except that compound (III) ($R_1$, $R_2$ = —$COCHCl_2$) used in Example 1 was replaced with 4'-dibromoacetyl-4'-demethylepipodophyllotoxin-β-D-2,3-di-O-dibromoacetyl-4,6-O-ethylideneglucoside (III) ($R_1$, $R_2$ = —$COCHBr_2$).

Example 11

1 g of compound (III) ($R_1$, $R_2$ = —$COCHCl_2$) was dissolved in 20 ml of methanol and after adding 0.64 g of diethylamine, the solution was stirred at 25°C for 10 minutes. After completion of the reaction, the solvent was distilled off in vacuum. After adding 20 ml of chloroform, the residue was neutralized with 2N hydrochloric acid, washed with water, and dried over anhydrous magnesium sulfate. The solution was concentrated in vacuum to a volume of 10 ml to obtain 0.53 g (yield: 83.1%) of etoposide crystals.

Example 12

0.51 g (yield: 80.0%) of etoposide crystals were obtained by the same reaction as in Example 11 except that diethylamine used in Example 11 was replaced with 0.88 g of di-n-propylamine.

Example 13

1 g of compound (III) ($R_1$, $R_2$ = —$COCHCl_2$) was added to 20 ml of methanol in which 0.15 g of ammonia gas had been dissolved, and the mixture was stirred at 25°C for 30 minutes. After completion of the reaction, the solvent was distilled off in vacuum. After adding 10 ml of chloroform, the residue was recrystallized to obtain 0.54 g (yield: 84.7%) of etoposide crystals.

Comparative Example 2

0.40 g (yield: 62.7%) of etoposide crystals were obtained by the same reaction as in Example 11 except that methanol used in Example 11 was replaced with 20 ml of diethylamine.

Likewise, the reaction was effected by replacing methanol used in Example 11 with 20 ml of pyridine.

Preparation Example 1

Production of 4'-dichloroacetyl-4'-demethylepipodophyllotoxin-β-D-2,3-di-O-dichloroacetyl-4,6-O-ethylideneglucoside (III) ($R_1$, $R_2$ = —$COCHCl_2$)

(a) 4'-dichloroacetyl-4'-demethylepipodophyllotoxin (V) ($R_2$ = —$COCHCl_2$)

8 g of 4'-demethylepipodophyllotoxin (IV) was dissolved in 160 ml of acetone and, after 3.2 g of pyridine, the solution was cooled to −5 to −10°C. To this solution, 4.1 g of dichloroacetyl chloride was added dropwise over a period of 1.5 hours, and the solution was further stirred for 0.5 hour. The acetone was distilled off in vacuum, and the obtained solid was dissolved in 160 ml of 1,2-dichloroethane and washed with water. The 1,2-dichloroethane solution was dried over anhydrous magnesium sulfate, and then concentrated in vacuum to obtain 9.5 g (yield: 93.4%) of compound (V) ($R_2$ = —$COCHCl_2$).

m.p. 207—208°C

IR $\nu_{max}^{KBr}$ 3540, 1775, 1600, 1485, 1235, 1130 $cm^{-1}$

(b) 4,6-O-ethylidene-1-O-benzyloxycarbonyl-2,3-di-O-dichloroacetyl-β-D-glucopyranose (VIII) ($R_1$ = —$COCHCl_2$)

34.0 g of 4,6-O-ethylidene-1-O-benzyloxycarbonyl-β-D-glucopyranose (VII) was suspended in 340 ml of 1,2-dichloroethane and, after adding 23.7 g of pyridine, the suspension was cooled to 0 to 5°C. To this suspension, 32.4 g of dichloroacetyl chloride was added dropwise over a period of about one hour and further stirred for 0.5 hour. The reaction solution was washed with water, and the organic layer was dried over anhydrous magnesium sulfate and concentrated in vacuum to obtain 51.0 g (yield: 90%) of compound (VIII) ($R_1$ = —$COCHCl_2$).

m.p. 150—151°C

IR $\nu_{max}^{KBr}$ 1770, 1255, 1100, 820 $cm^{-1}$

(c) 4,6-O-ethylidene-2,3-di-O-dichloroacetyl-β-D-glucopyranose (VI) ($R_1$ = —$COCHCl_2$)

10.0 g of compound (VIII) ($R_1$ = —$COCHCl_2$) was dissolved in 50 ml of acetone and, after adding 1.0 g of palladium black, the solution was subjected to hydrogenolysis at −5 to −10°C and an elevated pressure. After completion of the reaction, the catalyst was filtered off, and the solvent was distilled off in vacuum. 17 ml of diisopropyl ether was added to the residue, and the mixture was cooled to 0°C and filtered by suction to obtain 7.3 g (yield: 95.9%) of compound (VI) ($R_1$ = —$COCHCl_2$).

m.p. 133—135°C

IR $\nu_{max}^{KBr}$ 3445, 1775, 1305, 1165, 1095, 1005, 815 $cm^{-1}$

(d) 4′-dichloroacetyl-4′-demethylepipodophyllotoxin-β-D-2,3-di-O-dichloroacetyl-4,6-O-ethylideneglucoside (III) ($R_1$, $R_2$ = —$COCHCl_2$)

3.0 g of compound (V) ($R_2$ = —$COCHCl_2$) was dissolved in 60 ml of 1,2-dichloroethane and, after adding 2.5 g of compound (VI) ($R_1$ = —$COCHCl_2$), the solution was cooled to −10°C. 1.1 g of boron trifluoride ethyl etherate was added thereto dropwise over a period of about 1.5 hour, and, after completion of the addition, the reaction mixture was further stirred for 0.5 hour. 0.8 g of pyridine was added thereto dropwise while the internal temperature was being kept at −5 to −10°C, and the reaction mixture was washed with water. The organic layer was dried over anhydrous magnesium sulfate and concentrated in vacuum, and the residue was recrystallized from methanol to obtain 4.4 g (yield: 81.4%) of compound (III) ($R_1$, $R_2$ = —$COCHCl_2$).

m.p. 207—208°C

IR $\nu_{max}^{KBr}$ 1880, 1610, 1490, 1240, 1130, 935, 820 $cm^{-1}$

Further, compound (III) was obtained from 1.5 g of compound (V) and 5.0 g of compound (VI) by the same reaction and treatment as those described above.

Comparative Example 1

4′-Dibromoacetyl-4′-demethylepipodophyllotoxin-β-D-2,3-di-O-dibromoacetyl-4,6-O-ethylideneglucoside (III) ($R_1$, $R_2$ = —$COCHBr_2$)

(a) 4′-dibromoacetyl-4′-demethylepipodophyllotoxin (V) ($R_2$ = —$COCHBr_2$)

5.0 g of 4′-demethylepipodophyllotoxin was dissolved in 150 ml of 1,2-dichloroethane. After adding 1.5 g of pyridine, the solution was cooled to −5 to 10°C. To this solution, 3.8 g of dibromoacetyl chloride was added dropwise over a period of about 1.5 hours, and the mixture was further stirred for 0.5 hour. The reaction solution was washed with water, and the organic layer was dried over anhydrous magnesium sulfate and concentrated in vacuum to a volume of 50 ml to obtain a 1,2-dichloroethane solution of compound (V) ($R_2$ = —$COCHBr_2$).

(b) 4,6-O-ethylidene-1-O-benzyloxycarbonyl-2,3-di-O-dibromoacetyl-β-D-glucopyranose (VIII) ($R_2$ = —$COCHBr_2$)

5.1 g of 4,6-O-ethylidene-1-O-benzyloxycarbonyl-β-D-glucopyranose (VII) was suspended in 51 ml of 1,2-dichloroethane. After adding 3.6 g of pyridine, the suspension was cooled to 0 to 5°C. To this suspension, 7.8 g of dibromoacetyl chloride was added dropwise over a period of about one hour, and the mixture was further stirred for 30 minutes. Then, the reaction solution was washed with water, and the organic layer was dried over anhydrous magnesium sulfate and concentrated in vacuum to a volume of 25 ml to obtain a 1,2-dichloroethane solution of compound (VIII) ($R_1$ = —$COCHBr_2$).

(c) 4,6-O-ethylidene-2,3-di-O-dibromoacetyl-β-D-glucopyranose (VI) ($R_1$ = —$COCHBr_2$)

0.4 g of palladium black was added to 25 ml of the 1,2-dichloroethane solution of compound (VIII) ($R_1$ = —$COCHBr_2$) of (b), and the solution was hydrogenated at −10 to −5°C and an elevated pressure. After completion of the reaction, the catalyst was filtered off to obtain a 1,2-dichloroethane solution of compound (VI) ($R_1$ = —$COCHBr_2$).

(d) 4′-dibromoacetyl-4′-demethylepipodophyllotoxin-β-D-2,3-di-O-dibromoacetyl-4,6-O-ethylideneglucoside (III) ($R_1$, $R_2$ = —$COCHBr_2$)

25 ml of a 1,2-dichloroethane solution of compound (V) ($R_2$ = —$COCHBr_2$) was combined with 50 ml of a 1,2-dichloroethane solution of compound (VI) ($R_1$ = —$COCHBr_2$), and the combined solution was cooled to 10°C. 2.8 g of boron trifluoride ethyl etherate was added thereto dropwise over a period of about 1.5 hours. After completion of the addition, the reaction mixture was further stirred for 30 minutes. 2.0 g of pyridine was added thereto dropwise while the internal temperature was being kept at −5 to −10°C, and the reaction mixture was washed with water. The organic layer was concentrated in vacuum, and the residue was recrystallized from methanol to obtain compound (III) ($R_1$, $R_2$ = —$COCHBr_2$).

Preparation Example 2

4′-Dichloroacetyl-4′-demethylepipodophyllotoxin-β-D-2,3-di-O-trichloroacetyl-4,6-O-ethylideneglucoside (III) ($R_1$ = —$COCCl_3$, $R_2$ = —$COCHCl_2$)

(a) 4,6-O-ethylidene-1-O-benzyloxycarbonyl-2,3-di-O-trichloroacetyl-β-D-glucopyranose (VIII) ($R_1$ = —$COCCl_3$)

25 ml of 1,2-dichloroethane solution of compound (VIII) ($R_1$ = —$COCCl_3$) was obtained by the same reaction as in Comparative Preparation Example 1 except that dibromoacetyl chloride was replaced with trichloroacetyl chloride.

(b) 4,6-O-ethylidene-2,3-di-O-trichloroacetyl-β-D-glucopyranose (VI) ($R_1$ = —$COCCl_3$)

25 ml of a 1,2-dichloroethane solution of compound (VI) ($R_1$ = —$COCCl_3$) was obtained by the same reaction as in Comparative Preparation Example 1 (C) by using 25 ml of the solution obtained in (a).

(c) 4'-dichloroacetyl-4'-demethylepipodophyllotoxin-β-D-2,3-di-O-trichloroacetyl-4,6-O-ethylideneglucoside (III) ($R_1$ = —$COCCl_3$, $R_2$ = —$COCHCl_2$)

Compound (III) ($R_1$ = —$COCCl_3$, $R_2$ = —$COCHCl_2$) was obtained by reacting 25 ml of the solution obtained in (b) with 50 ml of a 1,2-dichloroethane solution containing compound (V) ($R_2$ = —$COCHCl_2$) obtained in Preparation Example 1(a) in the same manner as in Preparation Example 1(d).

## Claims

1. A process for producing etoposide represented by the formula (I):

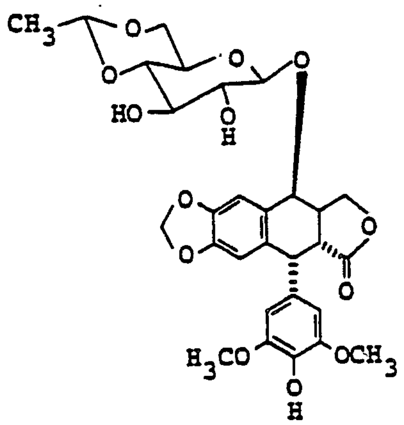

(I)

which comprises reacting a 4'-halogenoacetyl-4'-demethylepipodophyllotoxin-β-D-2,3-di-O-halogenoacetyl-4,6-O-ethylideneglucoside represented by the general formula:

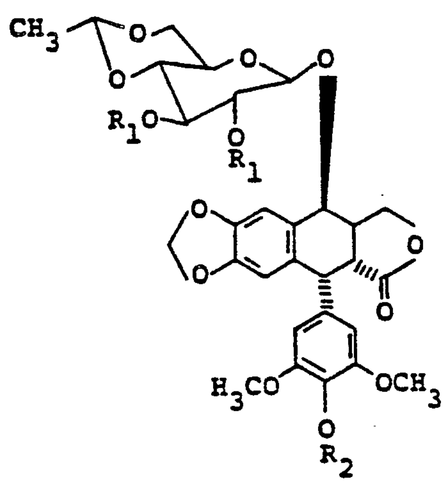

(III)

wherein $R_1$ and $R_2$, which may be the same or different, each represent —$COCHCl_2$ or —$COCCl_3$

(a) with an alcohol in the presence of a tri($C_1$—$C_4$ alkyl)amine, a pyridine or an organic carboxylic acid salt or

(b) with an amine and/or ammonia in an alcohol solvent, thereby to remove the halogenoacetyl groups so as to obtain etoposide of the formula (I).

2. A process according to claim 1, wherein the compound of formula (III) is reacted with a $C_1$—$C_4$ alcohol having from 1 to 3 hydroxyl groups, an aminoalcohol having from 1 to 3 $C_1$—$C_4$ hydroxyalkyl groups bonded to the nitrogen atom thereof, a mono- or di-($C_1$—$C_6$ alkyl)amine, a $C_4$ or $C_5$ cyclic amine which may contain at least one oxygen atom, an aliphatic diamine and/or ammonia.

3. A process according to claim 1 or 2, wherein said organic carboxylic acid salt is (1) an alkali metal salt, alkaline earth metal salt or ammonium salt of a $C_1$—$C_4$ aliphatic carboxylic acid, (2) a metal salt or ammonium salt of an aromatic carboxylic acid or (3) an ammonium salt- or metal salt-form weakly acidic cation exchange resin having carboxyl groups as exchangeable groups.

4. A process according to claim 3, wherein said organic carboxylic acid salt is an ammonium salt of a $C_1$—$C_3$ saturated fatty acid.

5. A process according to any one of claims 1 to 4, wherein the amount of said organic carboxylic acid salt is from 5 to 100 w/w % based on the compound of formula (III).

6. A process according to any one of the preceding claims, wherein the compound of formula (III) is reacted with methanol or ethanol.

7. A process according to claim 6, wherein the reaction is effected in the presence of ammonium acetate or ammonium formate.

8. A process according to any one of the preceding claims, wherein at least one equivalent of said alcohol or from 1 to 3 equivalents of said amine or ammonia is used per equivalent of the compound of formula (III).

9. A 4'-halogenoacetyl-4'-demethylepipodophyllotoxin-β-D-2,3-di-O-halogenoacetyl-4,6-O-ethylidene-glucoside represented by the formula (III):

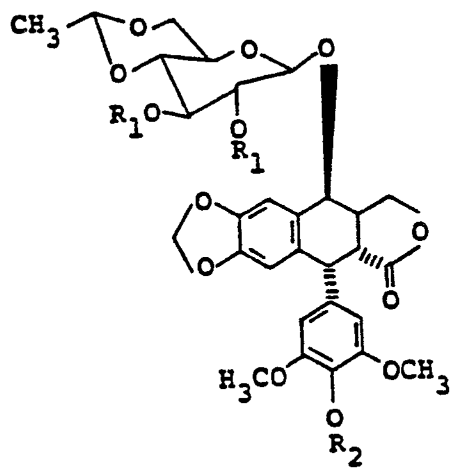

(III)

wherein $R_1$ and $R_2$, which may be the same or different, each represent $-COCHCl_2$ or $-COCCl_3$.

10. A process for the preparation of a compound of formula (III) as claimed in claim 9, which process comprises reacting 4'-demethylepipodophyllotoxin of the formula (IV):

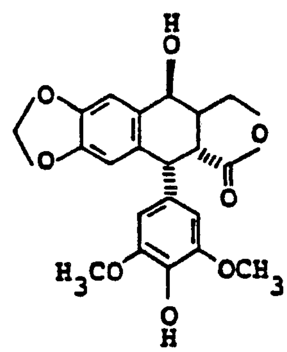

(IV)

with a dichloro- or trichloro-acetyl halide in an inert solvent, and condensing the resulting 4'-chloroacetyl-4'-demethylepipodophyllotoxin of the formula (V):

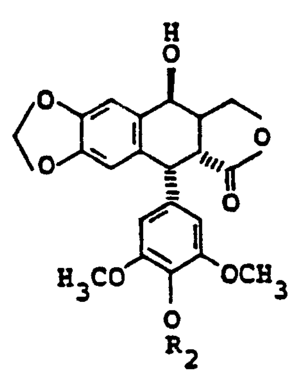

(V)

wherein $R_2$ represents $-COCHCl_2$ or $-COCCl_3$ with a 4,6-O-ethylidene-2,3-di-O-halogenoacetyl-β-D-glucopyranose of the formula (VI):

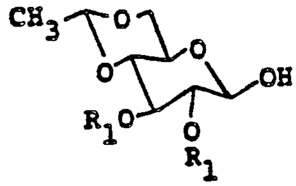

(VI)

(VI)

wherein $R_1$ represents $-COCHCl_2$ or $-COCCl_3$ at a temperature lower than 0°C in an inert solvent and in the presence of boron trifluoride ethyl etherate.

**Patentansprüche**

1. Verfahren zur Herstellung von Etoposide der Formel (I)

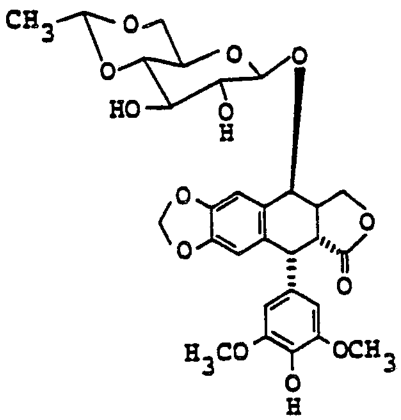

(I)

umfassend die Umsetzung eines 4-Halogenacetyl-4′-demethylepipodophyllotoxin-β-D-2,3-di-O-halogenacetyl-4,6-O-ethylidenglucosids der allgemeinen Formel (III):

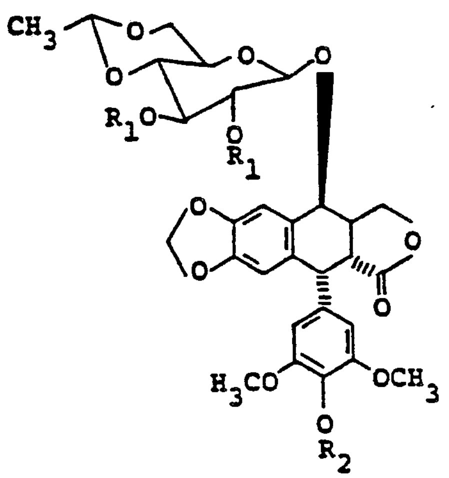

(III)

worin $R_1$ und $R_2$, die gleich oder verschieden sein können, jeweils $-COCHCl_2$ oder $-COCCl_3$ bedeuten,

(a) mit einem Alkohol in Gegenwart eines Tri($C_1$—$C_4$-alkyl)amins, Pyridin oder organischen Carbonsäuresalzes oder

(b) mit einem Amin und/oder Ammoniak in einem Alkohollösungsmittel,

um hierdurch die Halogenacetylgruppen zu entfernen, um so Etoposide der Formel (I) zu erhalten.

2. Verfahren nach Anspruch 1, wobei die Verbindung der Formel (III) mit einem $C_1$—$C_4$-Alkohol mit 1 bis 3 Hydroxylgruppen, einem Aminoalkohol mit 1 bis 3 $C_1$—$C_4$-Hydroxyalkylgruppen, die an dessen Stickstoffatom gebunden sind, einem Mono- oder Di-($C_1$—$C_6$-alkyl)amin, einem $C_4$ oder $C_5$-zyklischen Amin, das mindestens ein Sauerstoffatom enthalten kann, einem aliphatischen Diamin und/oder Ammoniak umgesetzt wird.

3. Verfahren nach Anspruch 1 oder 2, wobei das organische Carbonsäuresalz (1) ein Alkalimetallsalz, Erdalkalimetallsalz oder Ammoniumsalz einer $C_1$—$C_4$-aliphatischen Carbonsäure, (2) ein Metallsalz oder

Ammoniumsalz einer aromatischen Carbonsäure oder (3) eine Ammoniumsalz- oder Metallsalz-Form eines schwach sauren Kationenaustauschharzes mit Carboxylgruppen als austauschbaren Gruppen, ist.

4. Verfahren nach Anspruch 3, wobei das organische Carbonsäuresalz ein Ammoniumsalz einer $C_1$—$C_3$-gesättigten Fettsäure ist.

5. Verfahren nach einem der Ansprüche 1 bis 4, wobei die Menge des organischen Carbonsäuresalzes 5 bis 100 w/w-%, bezogen auf die Verbindung der Formel (III), beträgt.

6. Verfahren nach einem der vorangehenden Ansprüche, wobei die Verbindung der Formel (III) mit Methanol oder Ethanol umgesetzt wird.

7. Verfahren nach Anspruch 6, wobei die Umsetzung in Gegenwart von Ammoniumacetat oder Ammoniumformiat durchgeführt wird.

8. Verfahren nach einem der vorangehenden Ansprüche, wobei mindestens ein Äquivalent des Alkohols oder 1 bis 3 Äquivalente des Amins oder Ammoniaks pro Äquivalent der Verbindung der Formel (III) verwendet werden.

9. 4'-Halogenacetyl-4'-demethylepipodophyllotoxin-β-D-2,3-di-O-halogenacetyl-4,6-O-ethylidenglucosid der Formel (III):

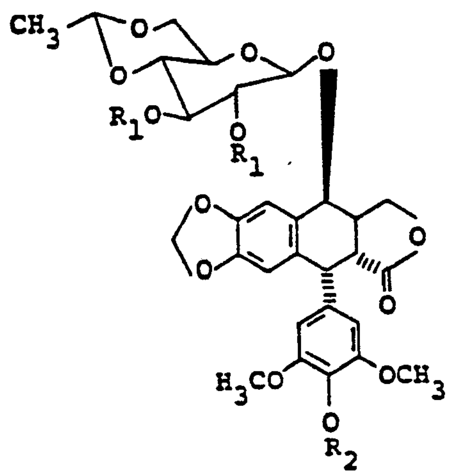

(III)

worin $R_1$ und $R_2$, die gleich oder verschieden sind können, jeweils $—COCHCl_2$ oder $—COCCl_3$ bedeuten.

10. Verfahren zur Herstellung einer Verbindung der Formel (III) gemäß Anspruch 9, umfassend die Umsetzung von 4'-Demethylepipodophyllotoxin der Formel (IV):

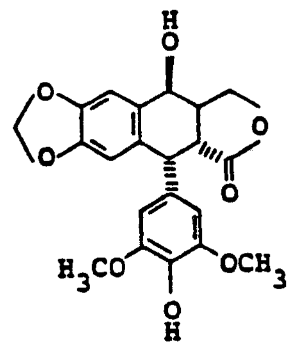

(IV)

mit einem Dichlor- oder Trichloracetylhalogenid in einem inerten Lösungsmittel und das Kondensieren des resultierenden 4'-Chloracetyl-4'-demethylepipodophyllotoxins der Formel (V):

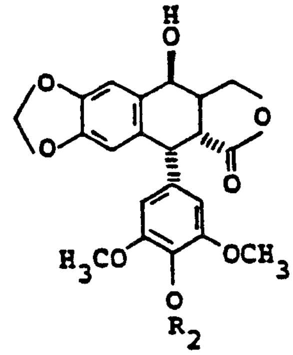

(V)

worin $R_2$ —$COCHCl_2$ oder —$COCCl_3$ bedeutet, mit einer 4,6-O-Ethyliden-2,3-di-O-halogenacetyl-β-D-glucopyranose der Formel (VI):

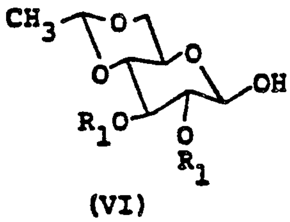

(VI)

(VI)

worin $R_1$ —$COCHCl_2$ oder —$COCCl_3$ bedeutet, bei einer Temperatur unterhalb 0°C in einem inerten Lösungsmittel und in Gegenwart von Bortrifluoridethyletherat.

**Revendications**

1. Procédé de production d'étoposide représenté par la formule (I):

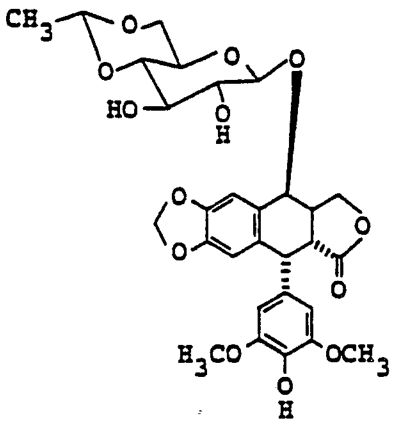

(I)

dans lequel on fait réagir un 4'-halogénoacétyl-4'-déméthylépipodophyllotoxine-β-D-2,3-di-O-halogénoacétyl-4,6-O-éthylidèneglucoside représenté par la formule générale:

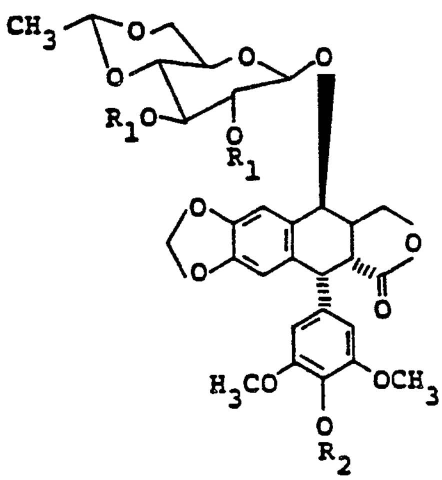

(III)

où $R_1$ et $R_2$, qui peuvent être identiques ou différents représentent chacun —$COCHCl_2$ ou —$COCCl_3$

(a) avec un alcool en présence d'une tri(alcoyle en $C_1$ à $C_4$)amine, d'une pyridine ou d'un sel d'acide carboxylique organique ou

(b) avec une amine et/ou de l'ammoniaque dans un solvant alcool, pour enlever ainsi les groupes halogénoacétyle de manière à obtenir l'étoposide de formule (I).

2. Procédé selon la revendication 1, dans lequel on fait réagir le composé de formule (III) avec un alcool en $C_1$ à $C_4$ ayant de 1 à 3 groupes hydroxyle, un aminoalcool ayant de 1 à 3 groupes hydroxyalcoyle en $C_1$ à $C_4$ reliés à son atome d'azote, une mono- ou di-(alcoyle en $C_1$ à $C_6$)amine, une amine cyclique en $C_4$ ou $C_5$ qui peut contenir au moins un atome d'oxygène, une diamine aliphatique et/ou de l'ammoniaque.

3. Procédé selon la revendication 1 ou 2, dans lequel ledit sel d'acide carboxylique organique est (1) un sel de métal alcalin, un sel de métal alcalino-terreux ou un sel d'ammonium d'un acide carboxylique aliphatique en $C_1$ à $C_4$, (2) un sel de métal ou un sel d'ammonium d'un acide carboxylique aromatique ou (3) une forme sel d'ammonium ou sel de métal de résine échangeuse de cations faiblement acide ayant des groupes carboxyle comme groupes échangeables.

4. Procédé selon la revendication 3, dans lequel ledit sel d'acide carboxylique organique est un sel d'ammonium d'un acide gras saturé en $C_1$ à $C_3$.

5. Procédé selon l'une quelconque des revendications 1 à 4, dans lequel la quantité dudit sel d'acide carboxylique organique est de 5 à 100% p/p sur la base du composé de formule (III).

6. Procédé selon l'une quelconque des revendications précédentes, dans lequel on fait réagir le composé de formule (III) avec du méthanol ou de l'éthanol.

7. Procédé selon la revendication 6, dans lequel la réaction est conduite en présence d'acétate d'ammonium ou de formiate d'ammonium.

8. Procédé selon l'une quelconque des revendications précédentes, dans lequel on utilise au moins un équivalent dudit alcool ou de 1 à 3 équivalents de ladite amine ou d'ammoniaque par équivalent du composé de formule (III).

9. 4'-Halogénoacétyl-4'-déméthylépipodophyllotoxine-β-D-2,3-di-O-halogénoacétyl-4,6-O-éthylidène-glucoside représenté par la formule (III):

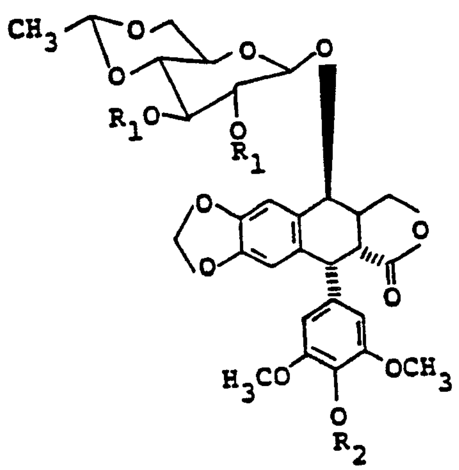

(III)

où $R_1$ et $R_2$, qui peuvent être identiques ou différents, représentent chacun $—COCHCl_2$ ou $—COCCl_3$.

10. Procédé de préparation d'un composé de formule (III) selon la revendication 9, procédé dans lequel on fait réagir de la 4'-déméthylépipodophyllotoxine de formule (IV):

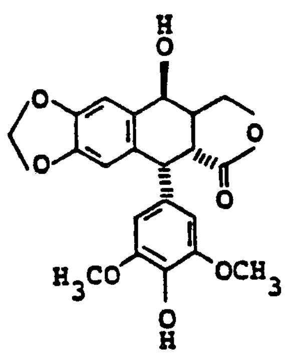

(IV)

avec un halogénure de dichloro- ou trichloro-acétyle dans un solvant inerte, et on condense la 4'-chloroacétyl-4'-déméthylépipodophyllotoxine résultante de formule (V):

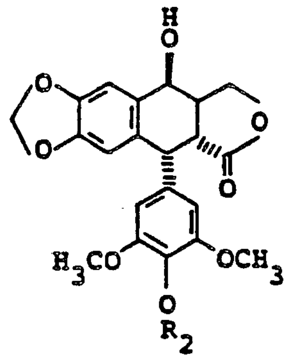

(V)

où $R_2$ représente $—COCHCl_2$ ou $—COCCl_3$ avec un 4,6-O-éthylidène-2,3-di-O-halogénoacétyl-β-D-glucopyranose de formule (VI):

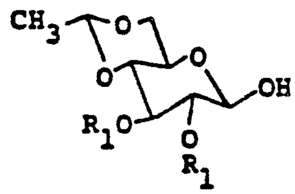

(VI)

(VI)

où $R_1$ représente $—COCHCl_2$ ou $—COCCl_3$ à une température inférieure à 0°C dans un solvant inerte et en présence d'éthyléthérate de trifluorure de bore.